Europäisches Patentamt

European Patent Office

Office européen des brevets

(11) Publication number: 0 297 789
A1

## (12) EUROPEAN PATENT APPLICATION

(21) Application number: 88305766.3

(22) Date of filing: 24.06.88

(51) Int. Cl.⁴: A61F 2/34

(30) Priority: 29.06.87 GB 8715176

(43) Date of publication of application:
04.01.89 Bulletin 89/01

(84) Designated Contracting States:
AT BE CH DE ES FR GB GR IT LI LU NL SE

(71) Applicant: HOWMEDICA INTERNATIONAL INC.
Shannon Industrial Estate
Shannon Co. Clare(IE)

(72) Inventor: Procter, Philip
Wilderwick Faris Lane
Woodham Weybridge Surrey.KT15 3DJ(GB)
Inventor: Jones Thomas Eric
Ardlahan
Kildimo Co.Limerick(IE)

(74) Representative: Bridge-Butler, Alan James et
al
G.F. REDFERN & CO. High Holborn House
52/54 High Holborn
London WC1V 6RL(GB)

(54) Method of making a prosthetic component and component made according to the method.

(57) A method of making a prosthetic component comprising one part which acts as a bearing liner (1) and another part which is a support or stiffener (2) for said liner, and which includes making one of the parts in a synthetic plastics material, using said first made part as a former to mould the other part from a different synthetic plastics material onto or into it, and arranging for the different synthetic plastics materials to bond together on moulding and/or to provide the former with means for keying the moulded part to it when moulding is completed.

FIG. 3

# METHOD OF MAKING A PROSTHETIC COMPONENT AND COMPONENT MADE ACCORDING TO THE METHOD.

This invention relates to a method of making a prosthetic component and a component made according to the method.

It is known to provide reinforced acetabular cups either pre-assembled or to be assembled by the surgeon immediately prior to use. Such cups of both types are known to be made from ultra high molecular weight polyethylene for the liner and cobalt-chrome alloys, titanium, or stainless steel for the stiffener or shell. Cups with such a reinforcement shell are made to provide additional strength as it is known that cups made merely from a synthetic plastics material tend to distort in use and lead to subsequent loosening. It is also known that metallic reinforcements are much stiffer than human bone and tend to lead to bone atrophy and thus loosening as a sequel. A cup with a metal reinforcing stiffener is shown, for example, in European Patent Application No. 84304092.4.

An alternative arrangement is to reinforce the shell to combat distortion by cold flow has been to reinforce the entire UHMWPE cup component with chopped carbon fibres. It is however known that all the rubbing artifical joint surfaces produce wear debris, and in the case where the rubbing surfaces are made of polymers intimately mixed with carbon fibres, such debris will include carbon fibre particles. It is further known, from experience with carbon fibre soft tissue replacements, that such debris may migrate away from the inplant site and into softer tissues and bodily organs.

In order to provide a shell stiffener which is not as stiff as the more conventional UHMWPE lined metal backed cups, and match as closely as possible the stiffness of living human bone it has been suggested, for example as in European Patent Application No. 84304092.4 to provide a stiffener made from carbon fibre or carbon fibre impregnated plastics material.

The present invention is intended to provide a method of making a prosthetic component having a liner and shell stiffener or support which can be utilised to produce a construction in which the bearing liner will not distort as excessively as the more conventional UHMWPE liner but which will have a support which is not as stiff as the more conventional UHMWPE lined metal backed components and which can utilise, for example, a synthetic plastics material support or stiffener. Thus the prosthetic component can match as closely as possible the stiffness of living human bone or, if the component is to be used with cement to match the reinforcement with the bone cement. The construction is therefore aimed at producing a component which will combat prosthetic component loosening.

According to the present invention a method of making a prosthetic component comprising one part which acts as a bearing liner and another part which is a support or stiffener for said liner includes making one of the parts in a synthetic plastics material, using said first made part as a former to mould the other part from a different synthetic plastics material onto or into it, and arranging for the different synthetic plastics materials to bond together on moulding and/or to provide the former with means for keying the moulded part to it when moulding is completed.

If desired the method may include forming a bearing surface on the bearing liner after moulding the two parts together.

It will be appreciated that the bearing liner and support or stiffener must be made from materials which are bio-compatible.

Preferably the support or stiffener is made from a material which, when moulding is completed, has a higher modulus of elasticity than the bearing liner.

The bearing liner can be made from ultra high molecular weight polyethylene, polyacetal, or epoxy resin.

If desired the bearing liner material may be reinforced with fibres, for example polyester fibres.

The term "fibres" is used herein to refer not only to single elements but also to multiple filaments entwined together. The term "ultra high molecular weight polyethylene" is used herein to describe polyethylene resins having a molecular weight which is greater than 1.5 million.

The support or stiffener can be made from synthetic plastics material reinforced with fibres which can be carbon, glass, aramid fibres (sold under the Trade Mark KEVLAR) or aramatic polyester fibres (sold under the Trade Mark DACRON).

In one preferred embodiment the synthetic plastics material of the support or stiffener is polybutyleneterephthalate.

Preferably the support or stiffener is provided with short fibre reinforcement with substantially random orientation.

Thus, the short fibre reinforcement may be in the form of chopped carbon fibres present in an amount of up to 40% by weight and the mean fibre length can be in the range 100 to 10,000 micrometres, the mean fibre diameter being between 3 to 15 micrometres.

The support or stiffener can be arranged to have a material stiffness in the range 0.5 to 50 Giga Newtons per square metre and a thickness in the range of 1 to 50 millimetres.

The invention also includes a prosthetic component comprising one part which acts as a bearing liner and another part which is a support or stiffener for said liner or fixing cement made by the method set forth above.

The invention can be performed in various ways but one embodiment will now be described by way of example and with reference to the accompanying drawings in which :

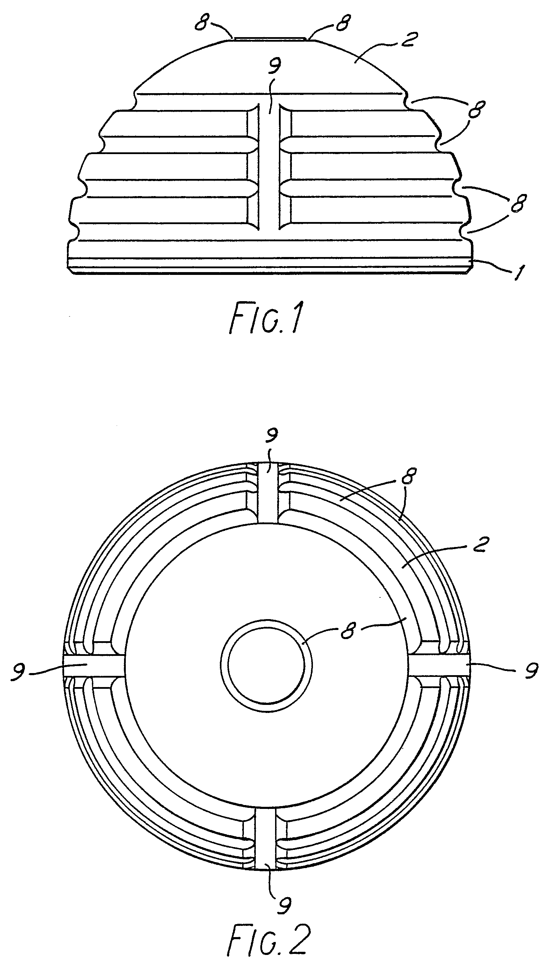

Figure 1 is a side elevation of an acetabular cup according to the invention;

Figure 2 is a plan view of the cup shown in Figure 1;

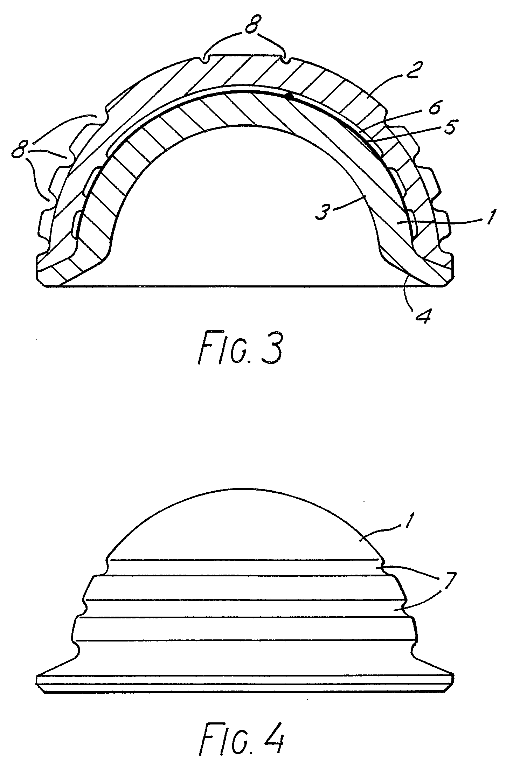

Figure 3 is a cross-sectional view of the acetabular cup shown in Figures 1 and 2; and

Figure 4 is a side elevation of the bearing liner after it has been made and before moulding the shell stiffener to it.

Loosening of acetabular cups and other prosthetic components in the bone is known and a clinically proven problem. Cemented components such as acetabular cups made entirely of plastic have been partially superceded by metal backed cups to reduce the likelihood of the cement breaking.

Cement free cups have a metal shell to avoid direct bone polyethylene contact which is biologically undesirable if it occurs. There is clinical evidence to suggest that these metal shells can be too stiff thereby creating another mechanism for loosening to occur. In order to reduce the stiffness of metal shells it is necessary either to change to a) low modulus materials, b) reduce the wall thickness, or c) put windows and holes in the metal shell. The lowest modulus metal is titanium which is still very high compared to bone cement and synthetic material with or without fibre reinforcement is another option. The difficulty with very thin shells is they are expensive to manufacture and windows and holes can create stress raisers in bone cement and can allow direct bone polyethylene contact in cement free designs.

A means of making an all plastics material cup stiffer without having a reinforcing shell has been to add fibre reinforcement to the complete body of the prosthesis. However, ultra high molecular weight polyethylene is shown to be very acceptable as a bearing surface and changing this material, or adding fibres to it introduces new unknown, unproven variables although the addition of such fibres may become acceptable.

The object of the present method is to produce an acceptable prosthetic component which has a reasonably close match of stiffness to the cement fixation mantle and/or is acceptable to the bone if a mechanical fixation means is used.

As shown in the drawings the prosthetic component according to the present invention is in the form of an acetabular cup which comprises one part which acts as a bearing liner 1 and another part which acts as a stiffener or support 2. The liner 1 has a part spherical bearing surface 3 for the head of the hip prosthesis and the mouth of the part hemi-spherical bearing surface 3 is widened at 4. The liner 1 is provided with a polar x-ray wire 5 which is located in a polar groove 6.

The support or stiffener 2 is moulded onto the liner 1 in a manner to be described so that the polar groove 6 is invested by the moulded shell stiffener thus forming an anti-rotation lock between the liner and the stiffener.

The liner 1 is provided with grooves 7 to provide a mechanical interlock between the liner and the shell stiffener when moulding is completed.

The outer surface of the shell stiffener 2 is also provided with circular grooves 8 and radial grooves 9 to assist in stabilizing and securing the shell stiffener to a layer of surgical cement when the cup is placed in the acetabular socket. It will be appreciated from the above that this particular type of cup is intended for cement location.

In order to make the prosthetic component shown in the Figures the bearing liner 1 is first made by any suitable means. Thus it could be made by machining or moulding and can be made from any suitable material, for example ultra-high molecular weight polyethylene. The radial grooves 7 and any other grooves, undercuts, holes or recesses can be preformed into the external surface of the liner, these openings being filled by the external shell stiffener material when it is moulded.

The bearing surface 3 of this inner component need not necessarily be formed before adding the outer shell. Indeed, the inner bearing component can be drilled and tapped for the purpose of holding it in a mould. If the bearing surface is later machine formed the tapped hole can be removed.

With the bearing liner 1 suitably supported in a mould the outer support or stiffener 2 is moulded onto it. The material used for the outer shell is preferably a carbon fibre filled polybutylene-terephthalate material which it has been found provides suitable stiffness. The precise dimensions and particular material used being determined appropriately.

The grooves, undercuts, holes and recesses formed in the outer surface of the bearing liner ensure that there is a suitable keying between the material and the outer layer holds the polar x-ray wire 5 in place.

If desired the different synthetic plastics materials can be chosen so that the two layers bond together with or without mechanical keying but preferably the various grooves, undercuts, holes and recesses are provided to ensure positive mechanical interlock.

An alternative way of making the prosthetic component is to first appropriately manufacture the outer shell stiffener, providing suitable grooves, holes and undercuts on its inner surface and to then mould the bearing liner into it.

The outer shell can be made from any convenient biocompatable material with suitable elastic modulus, the range preferably being from 10 to 20 Giga Newtons per square metre.

As mentioned above the bearing surface 3 can be formed either prior to moulding or after the prosthetic component has been otherwise completed.

Although the invention is described with reference to a method of making an acetabular cup it can be used for many other prosthetic components, for example knee tibial trays or appropriate parts in other joins of the body and again it will also be appreciated that the bearing liner need not be concave but, for certain applications could be convex or as desired.

## Claims

1. A method of making a prosthetic component comprising one part which acts as a bearing liner and another part which is a support or stiffener for said liner, and which includes making one of the parts in a synthetic plastics material, using said first made part as a former to mould the other part from a different synthetic plastics material onto or into it, and arranging for the different synthetic plastics materials to bond together on moulding and/or to provide the former with means for keying the moulded part to it when moulding is completed.

2. A method as claimed in claim 1 which includes forming a bearing surface on the bearing liner after moulding the two parts together.

3. A method as claimed in claim 1 or claim 2 in which the support or stiffener is made from a material when moulding is completed has a higher modulus of elasticity than the bearing liner.

4. A method as claimed in claims 1-3 in which the bearing liner is made from ultra high molecular weight polyethylene, polyacetal or epoxy resin.

5. A method as claimed in claim 4 in which the bearing liner material is reinforced with fibres.

6. A method as claimed in claim 5 in which the reinforcing fibres are polyester fibres.

7. A method as claimed in any one of the preceding claims in which the support or stiffener is made from synthetic plastics material reinforced with fibres.

8. A method as claimed in claim 7 in which the reinforcing fibres are carbon, glass, aramid fibres or aramatic fibres.

9. A method as claimed in claim 7 in which the synthetic plastics material of the support or stiffener is polybutylene-terephthalate.

10. A method as claimed in any one of claims 3, 7, 8 or 9, in which the support or stiffener is provided with short fibre reinforcement with substantially random orientation.

11. A method as claimed in claim 10 in which the short fibre reinforcement is in the form of shaped carbon fibres present in an amount of up to 40% by weight, the mean fibre length being in the range 100 to 10,000 micrometres and the mean fibre diameter being between 3 to 15 micrometres.

12. A method as claimed in any one of the preceding claims in which the support or stiffener has a material stiffness in the range of 0.5 to 50 Giga Newtons per square metre and a thickness in the range of 1 to 50 millimetres.

13. A prosthetic component comprising one part which acts as a bearing liner and another part which is a support or stiffener for said liner made by the method set forth in any one of the preceding claims.

FIG.1

FIG.2

FIG. 3

FIG. 4

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | FR-A-1 107 877 (TIMMERMANS)<br>* Whole document * | 1,13 | A 61 F 2/34 |
| A | US-A-3 806 960 (WEBER)<br>* Figure 5; column 2, line 64 - column 3, line 5 * | 1,13 | |
| A | EP-A-0 208 578 (QUEVEAU)<br>* Claim 4; figure 5 * | 1,13 | |
| D,A | EP-A-0 153 523 (HOWMEDICA INTERNATIONAL INC.)<br>* Claim 1; figures * | 1,13 | |

TECHNICAL FIELDS
SEARCHED (Int. Cl.4)

A 61 F 2/00

The present search report has been drawn up for all claims

| Place of search | Date of completion of the search | Examiner |
|---|---|---|
| BERLIN | 30-09-1988 | KANAL P K |

EPO FORM 1503 03.82 (P0401)